# EUROPÄISCHE PATENTSCHRIFT

(11) **EP 3 060 177 B1**
(45) Veröffentlichungstag und Bekanntmachung des Hinweises auf die Patenterteilung: **01.01.2020**
(21) Anmeldenummer: 14784251.2
(22) Anmeldetag: 16.10.2014
(51) Int. Cl.: A61F 2/90, A61F 2/07, A61F 2/89, A61F 2/915, A61F 2/06

(54) **GEFÄSSIMPLANTAT MIT BEREICHEN UNTERSCHIEDLICHER RADIALKRAFT**
VASCULAR IMPLANT HAVING PORTIONS OF DIFFERENT RADIAL FORCE
IMPLANT VASCULAIRE PRÉSENTANT DES ZONES DE FORCE RADIALE DIFFÉRENTE

(30) Priorität: 21.10.2013 DE 102013111593
(43) Veröffentlichungstag der Anmeldung: 31.08.2016
(73) Patentinhaber: JOTEC GmbH, 72379 Hechingen (DE)
(72) Erfinder: HAUSER, Berthold, 72393 Burladingen (DE)
(74) Vertreter: Witte, Weller & Partner Patentanwälte mbB
(86) Internationale Anmeldenummer: PCT/EP2014/072182
(87) Internationale Veröffentlichungsnummer: WO 2015/059019

(56) Entgegenhaltungen:
- EP-A1- 0 800 801
- EP-A1- 2 174 623
- WO-A2-01/93783
- DE-A1- 19 913 978
- US-A- 5 938 697
- US-A1- 2004 098 099
- US-A1- 2004 148 005

## Beschreibung

Die vorliegende Erfindung betrifft ein Gefäßimplantat zur Implantation in Gefäß eines Patienten, wobei das Gefäßimplantat von einem komprimierten Zustand in einen expandierten Zustand überführbar ist, mit einem hohlzylindrischen Grundkörper mit einer Längsrichtung sowie mit einem ersten und einem zweiten Ende und ersten und zweiten Endbereichen, und einem Grundkörperlumen, das sich vom ersten bis zum zweiten Ende erstreckt, wobei der hohlzylindrische Grundkörper durch ein röhrchenförmiges Gittergerüst gebildet wird.

Derartige so genannte verzweigte Gefäßimplantate sind im Stand der Technik bekannt. Solche Gefäßimplantate, die auch als endovaskuläre Gefäßstents/-stentgrafts bezeichnet werden, werden allgemein zur Offenhaltung von Gefäßen, insbesondere Blutgefäßen, sowie zur Behandlung von Aneurysmen in Arterien implantiert. Dabei versteht man unter einem Aneurysma eine Ausweitung oder Aussackung eines arteriellen Blutgefäßes infolge angeborener oder erworbener Wandveränderungen. Die Aussackung kann dabei die Gefäßwand als Ganzes erfassen oder, wie bei dem sogenannten falschen Aneurysma bzw. der sogenannten Dissektion, es tritt Blut aus dem Lumen des Gefäßes zwischen die Gefäßwandschichten und schert diese auseinander. Die Nichtbehandlung eines Aneurysmas kann im fortgeschrittenen Stadium zu einer Ruptur der Arterie führen, mit der Folge, dass der Patient innerlich verblutet.

Zur Behandlung von Aneurysmen bzw. von zu verschließen drohenden Gefäßen wird daher das betroffene Gefäß durch Implantation eines Stents/Stentgrafts stabilisiert, um eine Ruptur des Gefäßes zu vermeiden bzw. allgemein die Offenhaltung des Gefäßes zu gewährleisten.

Im Allgemeinen wird unter einem "Stent" dabei eine Gefäßstütze bzw. ein medizinisches Implantat in Form eines kleinen Gittergerüsts in Röhrchenform aus Metall oder Kunststofffasern verstanden, bzw. eine jede solche Struktur, die einer Prothese eine Expansionskraft und/oder eine stützende Funktion verleiht. Bei einem "gecoverten Stent" sind dabei die Maschen des Gittergerüsts überwiegend durch ein Implantatmaterial bedeckt; dadurch wird eine röhrchenförmige Struktur (ein "Stentgraft") geschaffen, die aus einem Implantatmaterial besteht, die von einem (Metall-)Gerüst (dem Stent-Teil) getragen wird.

Die zur Offenhaltung von Gefäßen bzw. zur Behandlung von Aneurysmen verwendeten Stents/Stengrafts bestehen dabei im Allgemeinen aus einem röhrchenförmigen Metallrahmen, dessen Mantelfläche mit einer Textil- oder Polymerfolie abgedeckt sein kann, so dass sich ein hohlzylindrischer Körper ergibt. Zur Implantation wird der Stent - bspw. mittels einer den Stent umgebenden und komprimierenden Hülle - radial zusammengedrückt, so dass sich seine Querschnittsfläche deutlich verringert. Der Stent/Stentgraft wird dann mit Hilfe eines Einführsystems in den Bereich der zu behandelnden Stelle bzw. des Aneurysmas gebracht, wo der Stent freigesetzt wird. Aufgrund der Federwirkung des Metallrahmens expandiert der Stent wieder in seine ursprüngliche Form und spannt dabei seine Mantelfläche auf, die sich proximal und distal von dem Aneurysma bzw. der offen zu haltenden Stelle innen in dem Blutgefäß verklemmt. Auf diese Weise fließt das Blut jetzt durch den Stent/Stentgraft, wodurch eine weitere Belastung der Aussackung verhindert und die Offenhaltung des Gefäßes gewährleistet wird.

Die Expansion des Metallrahmens kann einerseits durch die Verwendung von selbstexpandierendem Metall, wie bspw. Nitinol, bewirkt werden, oder aber durch Einsatz eines Dilatations-Ballons, der von innen in den Metallrahmen eingeführt wird und durch dessen Dilatation auch der Metallrahmen expandiert wird.

Oftmals zweigen an der Stelle des Gefäßes, an welcher ein solcher Stent/Stentgraft bzw. eine solche Gefäßprothese eingeführt werden soll, Seiten-Blutgefäße ab, weshalb bei Einbringung des Gefäßimplantats dann die Gefahr besteht, dass diese Seitengefäße durch die Gefäßprothese im Hauptgefäß bzw. durch das blutdichte Prothesenmaterial von der Blutzufuhr abgeschnitten werden. Daher weisen Gefäßprothesen in diesen Bereichen oftmals Öffnungen, sog. "Fenestrierungen", im Mantel- bzw. Prothesenmaterial auf, um das durch die Gefäßprothese fließende Blut durch diese Öffnungen auch in die vom Gefäß abzweigenden Seiten-Gefäße zu lenken. Dadurch wird auch eine Blutversorgung der Körperbereiche, die von den Seite-Gefäßen versorgt werden, gewährleistet.

In vielen Fällen weisen die an solchen abzweigenden Bereichen einzubringenden Gefäßimplantate bzw. Stents/Stentgrafts nicht nur Fenestrierungen, sondern vom Gefäßimplantatgrundkörper abzweigende Seitenäste auf, die im Aneurysmasack oberhalb des abzweigenden Gefäßes freigesetzt werden und bspw. als Landungszone für einen weiteren Stentgraft dienen, der zur Überbrückung des Aneurysmas in den Seitenast und das abzweigende Gefäß implantiert wird. Dadurch wird zusätzlich sichergestellt, dass auch die Seiten-Gefäße mit Blut versorgt werden.

Kritische Stellen im Gefäßsystem des Menschen stellen insbesondere sich aufzweigende Gefäße dar, bei welchen es wichtig ist, die Versorgung der abzweigenden Gefäße stabil zu gewährleisten. Die gegenwärtig im Stand der Technik bekannten und erhältlichen Gefäßimplantate mit Seitenästen sind üblicherweise durch zwischen den Stentfedern bzw. Mahschen des Stent-Gittergerüsts angesetzte Seitenäste bewirkt.

Die abgehenden Gefäße werden dabei anatomisch bedingt nach proximal - d.h. in die vom Hauptgefäß wegführende Richtung - immer dünnwandiger, und dadurch empfindlich und verletzlich. Mit den gegenwärtig eingesetzten, "einstückigen", gleichmäßig starken/steifen Gefäßimplantaten ist es nicht möglich, diesen unterschiedlichen Gefäßgegebenheiten und -anforderungen gerecht zu werden, so dass insbesondere bei kleineren abzweigenden Gefäßen die Gefahr besteht, dass diese aufgrund der durch den Stent ausgeübten Druckbelastung reißen.

Im Stand der Technik wird dieser Problematik auch durch den Einsatz zweier unterschiedlich weicher/flexibler Stents/Stentgrafts begegnet, die nacheinander in die betroffenen Gefäße implantiert werden. Diese Modelle haben jedoch den Nachteil, dass sie zeit- und materialaufwändig einzubringen sind.

Die EP 2 174 623 A1 offenbart einen Stent mit verschiedenen Abschnitten, mit einer sich über die Länge des Stents graduell variierenden radialen Steifigkeit, wobei die radiale Steifigkeit am Ende des Stents höher ist als in mittleren Stentbereichen.

Ferner offenbart die EP 0 800 801 A1 einen expandierbaren Stent mit einer offenen Gitterstruktur, die derart ausgebildet ist, dass ein Endabschnitt einen dickeren Durchmesser und eine entsprechend größere radiale Steifigkeit besitzt.

Aufgabe der vorliegenden Erfindung ist es daher, eine Gefäßprothese bereitzustellen, mit welcher die oben geschilderten Nachteile der im Stand der Technik bekannten Gefäßprothesen überwunden werden können, und mit welcher den durch die jeweiligen Gefäßgegebenheiten bestehenden Anforderungen optimal begegnet werden kann.

Erfindungsgemäß wird diese Aufgabe durch ein wie in den Ansprüchen definiertes Gefäßimplantat gelöst, das zumindest einen ersten härteren Bereich mit einer Radialkraft und einer Steifigkeit aufweist, die höher ist als die Radialkraft und Steifigkeit zumindest eines zweiten weicheren Bereichs ist, welcher zum ersten härteren Bereich benachbart ist, wobei das Gefäßimplantat ferner über seine Längsrichtung einen kontinuierlichen Übergang von der höheren Steifigkeit und Radialkraft im ersten härteren Bereich zur niedrigeren Steifigkeit und Radialkraft im zweiten weicheren Bereich aufweist.

Die der Erfindung zugrunde liegende Aufgabe wird auf diese Weise vollkommen gelöst. Das erfindungsgemäße Gefäßimplantat weist Bereiche mit unterschiedlicher Steifigkeit/Radialkraft auf; entsprechend dieser Bereiche kann es mit Rücksicht auf die jeweiligen Gegebenheiten des zu behandelnden Gefäßeses in dieses eingebracht werden, und zwar derart, dass der weichere, flexiblere Bereich mit geringerer Radialkraft und Steifigkeit in einem ersten Gefäßabschnitt zu liegen kommt, der dünner und empfindlicher ist als ein zweiter Bereich, der proximal vom ersten Gefäßabschnitt liegt und damit weniger dünn und empfindlich ist als der erste Gefäßabschnitt.

Vorliegend wird dabei unter "Radialkraft" die Kraft verstanden, mit der das Gefäßimplantat nach außen, d.h. von einer durch das Gefäßimplantat gedachten Längsachse weg, und in Richtung Gefäßwand, drückt. Durch diese Kraft stabilisiert das Gefäßimplantat das Gefäß und die auftretenden Kräfte werden von dem Gefäßimplantat aufgenommen. Dabei ist es wichtig, dass die Radialkraft des Gefäßimplantats an das zu behandelnde Gefäß derart angepasst ist, dass dieses zwar aufgeweitet wird, um den Blutfluss durch das Gefäß zu gewährleisten, dass sie aber andererseits nicht zu groß ist, da ansonsten die Gefahr von Gefäßwandverletzungen durch Überdehnung besteht.

Vorliegend wird allgemein unter "Stent" jede Vorrichtung oder eine Struktur bezeichnet, die einer Prothese eine Expansionskraft und/oder eine stützende Funktion verleiht.

Der Ausdruck "Stentgraft" soll vorliegend - wie auch im Stand der Technik - eine Prothese bedeuten, die einen bzw. mehrere Stent (oder Stentfedern) sowie ein damit verbundenes Implantat("graft")-Material aufweist, das ein Lumen durch zumindest einen Abschnitt der Prothese bildet.

Ferner wird vorliegend, sowie gemäß der im Stand der Technik verwendeten Nomenklatur, mit "proximal" diejenige Position, Richtung oder ein Abschnitt oder Ende einer Komponente des Gefäßimplantats bezeichnet, die/der am nächsten bzw. näher zum Herzen des zu behandelnden Patienten liegt als eine anderer Abschnitt oder Komponente.

Entsprechend wird vorliegend mit "distal" diejenige Position, Richtung oder ein Abschnitt oder Ende einer Komponente des erfindungsgemäßen Gefäßimplantats bezeichnet, die vom Herzen eines Patienten weiter/am weitesten entfernt ist/führt.

Entsprechend sind vorliegend die "proximale" und die "distale" Öffnung des Gefäßimplantats die Öffnungen, durch die hindurch der Blutfluss durch den hohlzylindrischen Körper der Gefäßimplantat gewährleistet wird: Wenn das erfindungsgemäße Gefäßimplantat in einem Blutgefäß wie beispielsweise der Aorta implantiert ist, fließt also das vom Herzen kommende Blut durch die proximale Öffnung des Gefäßimplantats, und verlässt das Gefäßimplantat durch deren distale Öffnungen.

Erfindungsgemäß ist das erste Ende das proximale Ende, und das zweite Ende das distale Ende, wobei der erste härtere Bereich im proximalen Endbereich gebildet ist, und der zweite weichere Bereich im distalen Endbereich. Erfindungsgemäß ist also das proximale Ende dasjenige Ende, das in Bezug auf den Verlauf des Gefäßes - näher zum Herzen liegt als das andere, das distale Ende, das vom Herzen weiter entfernt ist. Bei einer Einbringung des Gefäßimplantats an einer Abzweigung, also als von einem Hauptimplantat abzweigender Seitenast, bspw. über eine Fenestrierung im Hauptimplantat, liegt also das proximale Ende des erfindungsgemäßen Gefäßimplantats an der Abzweigung des Seitengefäßes vom Hauptgefäß, wo es aufgrund seiner höheren Steifigkeit und Radialkraft eine sichere Verbindung mit dem Hauptimplantat und eine sichere, da starke Abdichtung dem Gefäß gegenüber gewährleistet; das distale Ende mit der geringeren Radialkraft kommt im dünneren, empfindlicheren Seitengefäß zu liegen, wodurch dieses geschont wird, und Verletzungen vorteilhafterweise vermieden werden.

Gemäß einer bevorzugten Ausführungsform weist das erfindungsgemäße Gefäßimplantat zumindest zwei erste härtere Bereiche auf, die durch zumindest einen zweiten weicheren Bereich von einander getrennt sind. Diese Ausführungsform hat den Vorteil, dass sie bspw. bei Gefäßstellen eingesetzt werden kann, bei dem ein empfindlicher Bereich überbrückt werden muss, der nur mit wendig Radialkraft belastet werden darf.

Gemäß einer noch weiteren Ausführungsform weist das erfindungsgemäße Gefäßimplantat zumindest zwei zweite weichere Bereiche auf, die durch zumindest einen ersten härteren Bereich voneinander getrennt sind. Diese Ausführungsform hat den Vorteil, dass sie aufgrund des härteren mittleren Bereichs sicher und abdichtend im Gefäß verankert werden kann, während die Bereiche rechts und links davon für Gefäßbereiche vorgesehen sind, die aufgrund der speziellen Anatomie des Gefäßes oder der spezifischen, individuellen Gegebenheit (bspw. bei einem erkrankten Gefäßabschnitt) des Gefäßes nicht stark belastet werden dürfen.

Erfindungsgemäß weist das Gefäßimplantat weist zumindest einen Bereich auf, der ein mit dem Gittergerüst befestigtes Implantatmaterial aufweist.

Bei dieser Ausführungsform handelt es sich also um ein sogenanntes "gecovertes" Gefäßimplantat, das aus einem (Stent)-Gittergerüst und einem dieses zumindest teilweise bedeckende (Graft-)Material besteht. Derartige Gefäßimplantate werden bspw. bei Aneurysmen eingesetzt, um den Bereich einer Aussackung dichtend zu überbrücken.

Dabei ist bei einer bevorzugten Ausführung eines solchen gecoverten Gefäßimplantats vorgesehen, dass das Gefäßimplantat insgesamt mit dem Implantatmaterial gecovert ist, mithin also jeder Bereich des Gefäßimplantats ein mit dem Gittergerüst befestigtes Gefäßimplantatmaterial aufweist. Dabei versteht sich, dass die jeweils endständige Gitterstruktur, also das jeweils äußerste proximale und distale Ende zur Verankerung im Gefäß freie Gitterenden aufweisen kann.

Erfindungsgemäß ist das röhrchenförmige Gittergerüst aus hintereinander angeordneten Stentfedern gebildet. Im Falle der Ausbildung des Gittergerüsts durch hintereinander angeordnete Stentfedern, insbesondere aus Metalldrähten, sind diese vorzugsweise lediglich indirekt über das Prothesenmaterial, nicht aber untereinander verbunden, und weisen keine Stege oder sonstigen Kreuzungen der die Stentfedern bildenden Metalldrähte auf.

Dabei wird unter einer "Stentfeder", wie eingangs diskutiert, jedes einstückige, ringförmige Element verstanden, das sich aufgrund dessen Materials komprimieren lässt, und sich nach Entfernung des Kompressionsdrucks wieder federartig expandieren kann. Unter "mäanderförmig" wird vorliegen jeder schlingen- bzw. schleifenförmige Verlauf" der Stentfeder bzw. des Stentdrahtes verstanden, wobei jede Stentfeder einstückig, d.h. aus einem mäanderförmig umlaufenden Stentfederring gebildet ist.

Eine "mäanderförmig umlaufende einstückige Stentfeder" ist in diesem Zusammenhang vorliegend ein sich federartig expandierendes und komprimierbares ringförmiges Stentelement, das einen wellenartigen Umlauf besitzt, wobei sich Wellenberg und Wellental, die eine Phase bilden, abwechseln.

Erfindungsgemäß ist dabei ein Spitzbogen jeweils aus zwei Schenkeln und einem zwischen den Schenkeln liegenden Scheitelpunkt bzw. Tiefstpunkt gebildet.

Erfindungsgemäß ist das röhrchenförmige Gittergerüst aus hintereinander angeordneten und senkrecht zur Längsrichtung jeweils mäanderförmig umlaufenden einstückigen Stentfedern gebildet, die abwechselnd zum proximalen und distalen Ende des Grundkörpers und parallel zu dessen Längsrichtung weisende Spitzbögen aufweisen, wobei ein Spitzbogen aus jeweils einem Scheitelpunkt und zwei Schenkeln gebildet ist, wobei die Stentfedern im proximalen Endbereich jeweils mehr Spitzbögen aufweisen als die Stentfedern im distalen Endbereich.

Bei diesem Gefäßimplantat stehen die Stentringe bzw. Stentfedern nicht direkt miteinander in Kontakt, sie überschneiden sich also nicht oder sind auch sonst nicht durch bspw. Verbindungsstege mit einander verbunden, sondern bilden lediglich durch das Implantatmaterial, an welches die Stentfedern oder -ringe angebracht, bspw. angenäht werden, die röhrchenförmige Struktur aus. Dabei ist vorliegend mit "mehr Spitzbögen" gemeint, dass eine - im proximalen Endbereich - umlaufende Stentfeder in ihrem Umfang mehr Scheitel- bzw. Tiefstpunkte aufweist als eine andere, im distalen Endbereich umlaufende Stentfeder.

Dadurch, dass im proximalen Endbereich mehr Spitzbögen vorliegen als im distalen Endbereich, wird in dem proximalen Bereich eine erhöhte Radialkraft erreicht, wohingegen im distalen Bereich weniger Spitzbögen vorliegen, wodurch der Stent in diesem, also im distalen Bereich eine niedrigere Radialkraft aufweist.

Gemäß einer bevorzugten Ausführungsform weisen dabei die Schenkel der Spitzbögen im distalen Endbereich darüber hinaus eine Länge auf, die größer ist als die Länge der Schenkel der Spitzbögen im proximalen Endbereich. Dadurch wird erreicht, dass die Spitzbögen insgesamt höher bzw. tiefer sind, also der wellenförmige Verlauf einen "höheren" Ausschlag/Amplitude besitzt, was ebenfalls dazu beiträgt, dass im distalen Bereich eine niedrigere Radialkraft besteht.

In einer bevorzugten Ausführungsform des erfindungsgemäßen Gefäßimplantats ist das Implantatmaterial, vom proximalen Endbereich aus beginnend, mit dem Gittergerüst über eine Fläche verbunden, die ausgewählt ist aus mind. ca. 30%, 50% oder 100% der Gesamtfläche des Gefäßimplantats.

Gemäß einer bevorzugten Ausführungsform weist das Gefäßimplantat ein Material auf, das ausgewählt ist aus Nitinol oder Stahl.

Das Gittergerüst des erfindungsgemäßen Gefäßimplantats besteht dabei vorzugsweise aus einem Material mit Formgedächtnis, insbesondere aus Nitinol.

Gemäß einer weiteren bevorzugten Ausführungsform des erfindungsgemäßen Gefäßimplantats weist dieses Nitinol auf oder ist aus diesem gebildet, und kann von einem nicht expandierten in einen selbstexpandierten Zustand überführt werden, wobei das Gefäßimplantat im selbstexpandierten Zustand ein gegenüber dem distalen Ende aufgeweiteteres proximales Ende aufweist.

Gemäß einer anderen bevorzugten Ausführungsform des erfindungsgemäßen Gefäßimplantats weist dieses Stahl auf oder ist aus diesem gebildet, wobei das das proximale Ende über einen expandierbaren Ballon von einem nicht-expandierten Zustand in einen ballon-expandierten Zustand aufweitbar ist, und wobei das distale Ende weich ist.

Die gemäß den erfindungsgemäßen Ausführungsformen aufgeweiteten proximalen Enden haben den Vorteil, dass über diese eine feste Verankerung erreicht werden kann, sei es im Gefäß oder in einer Fenestrierung eines anderen Gefäßimplantats zur Ausbildung eines Seitenasts.

Daher ist gemäß einer weiteren bevorzugten Ausführungsform vorgesehen, dass das Gefäßimplantat zur Einbringung in ein von dem Gefäß abzweigendes Seitengefäß ausgebildet ist, wobei der proximale Endbereich vorgesehen ist, über eine Fenestrierung eines im Gefäß positionierten Hauptgefäßimplantats an der Abzweigung des Seitengefäßes positioniert zu werden, und der distale Endbereich im abzweigenden Seitengefäß positionierbar ist.

Dadurch wird vorteilhaft bewirkt, dass der distale Endbereich mit geringerer Radialraft und Steifigkeit in einem Abschnitt des Seitengefäßes positioniert wird, der aufgrund seiner Dünnwandigkeit empfindlicher ist als der Abschnitt des Seitengefäßes, der sich unmittelbar an der Abzweigung von einem Hauptgefäß befindet und noch dickwandiger aufgebaut ist.

Die Radialkraft wird in Newton gemessen und gibt die Kraft an, mit der das Gefäßimplantat einem Druck (bspw. einer Gefäßwand) entgegenwirken kann. Für die Radialkraftmessung kann das Gefäßimplantat bspw. zwischen einem verfahrbaren Stempel und einer festen Aufnahme deformiert werden. Das Gefäßimplantat kann für die Messung um einen Prozentsatz seines Durchmessers komprimiert werden, wobei ein Prozentsatz von 10, 20, 30, 40, oder 50 % bevorzugt ist. Vorzugsweise weist die Aufnahme dabei Form auf, die ausgewählt ist aus einer eckigen klammerförmigen Aufnahme, einer U-förmigen Aufnahme oder einer V-förmigen Aufnahme. Die durch den Stempel erzeugte Radialkraft des Gefäßimplantats wird mittels einer Kraftmessdose gemessen. Während des Messvorgangs wurde, um das Materialverhalten des Gefäßimplantats unter "in vivo"-Bedingungen abzubilden, dieses auf 37°C temperiert, was bspw. durch Einbringen der Messvorrichtung aus Stempel, Messdose (Newton) und Aufnahme inkl. Gefäßimplantat in ein entsprechend temperiertes Wasserbad erreicht werden kann.

Bei einer Aufnahme des Gefäßimplantats in einer eckigen oder V-förmigen klammerförmigen Aufnahme wird das Gefäßimplantat jeweils an drei Punkten fixiert; bei einer Aufnahme in einer U-förmigen Aufnahme wird das Gefäßimplantat über seinen halben Umfang und seine gesamte Länge fixiert.

Gemäß einer weiteren bevorzugten Ausführungsform ist das röhrchenförmige Gittergerüst des erfindungsgemäßen Gefäßimplantats ein gelaserter Nitinolstent und weist in dem zumindest einen härteren Bereich eine Radialkraft mit einem Wert von ca. zwischen 2,00 und 4,00 N (Newton) auf, und in dem zumindest einen weicheren zweiten Bereich weist die Radialkraft einen Wert von ca. zwischen 0,50 und 2,00 N (Newton). Dabei ist gemäß einer bevorzugten Ausführungsform vorgesehen, wenn das rörchenförmige Gittergerüst in dem zumindest einen härteren Bereich einen Wert für die Radialkraft ca. 3,00 N (Newton) aufweist, und in dem zumindest einen weicheren zweiten Bereich einen Wert von ca. 1,00 N (Newton).

Ferner ist gemäß einer anderen bevorzugten Ausführungsform bevorzugt, wenn das röhrchenförmige Gittergerüst des erfindungsgemäßen Gefäßimplantats ein geflochtener Nitinolstent ist und in dem zumindest einen härteren Bereich eine Radialkraft mit einem Wert von ca. zwischen 8,00 und 16,00 N (Newton) aufweist, und in dem zumindest einen weicheren zweiten Bereich weist die Radialkraft einen Wert von ca. zwischen 2,00 und 8,00 N (Newton).

Gemäß einer noch weiteren bevorzugten Ausführungsform ist das röhrchenförmige Gittergerüst des erfindungsgemäßen Gefäßimplantats ein gelaserter Stahlstent und weist in dem zumindest einen härteren Bereich eine Radialkraft mit einem Wert von ca. zwischen 5,00 und 10,00 N (Newton) auf, und in dem zumindest einen weicheren zweiten Bereich weist die Radialkraft einen Wert von ca. zwischen 1,25 und 5,00 N (Newton).

Dabei ist bevorzugt, wenn die Radialkraftmessung bei einer Komprimierung des Gefäßimplantats um 30% erfolgt, und weiter bevorzugt, wenn darüber hinaus die Messung in einer den Stent über drei Punkte fixierenden Aufnahme erfolgt, und noch weiter bevorzugt, wenn darüber hinaus die Messung bei ca. 37°C erfolgt.

Dabei soll der Begriff "ca." vorliegend und wie auch allgemein bedeuten, dass nicht nur der ganz exakte jeweils angegebene Wert gemeint sind, sondern auch übliche Messtoleranzen bei der Bestimmung der jeweiligen Werte.

Insgesamt kann das erfindungsgemäße Gefäßimplantat bzw. dessen hohlzylindrischer Grundköper in all seinen bevorzugten Ausführungsformen darüber hinaus über seine Gesamtlänge hinweg einen einheitlichen Durchmesser, oder aber unterschiedliche Durchmesser aufweisen.

Weitere Vorteile ergeben sich aus der nachstehenden Beschreibung vorteilhafter Ausführungsformen und den beigefügten Figuren.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den nachstehenden Figuren dargestellt und werden in Bezug auf diese im Folgenden näher beschrieben. Es zeigen:
Fig. 1: eine schematische, nicht-maßstabsgetreue Darstellung eines ersten Ausführungsbeispiels des erfindungsgemäßen Gefäßimplantats;
Fig. 2: eine schematische, nicht-maßstabsgetreue Darstellung eines nicht zur Erfindung gehörenden Gefäßimplantats;
Fig. 3 die schematische, nicht-maßstabsgetreue Darstellung einer in ein abzweigendes Gefäß eingebrachten Ausführungsform des erfindungsgemäßen Gefäßimplantat; und
Fig. 4: eine schematische Darstellung des Versuchsaufbaus zur Messung der Radialkraft eines Gefäßimplantats.

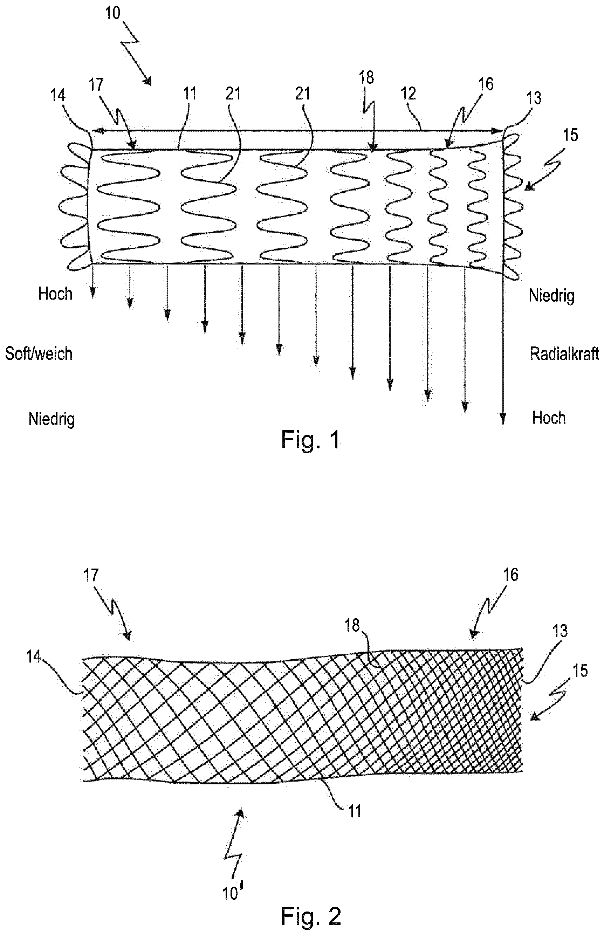

Fig. 1 zeigt eine nicht-maßstabsgetreue Darstellung einer Ausführungsform eines erfindungsgemäßen Gefäßimplantats 10, der einen hohlzylindrischen Grundkörper 11, sowie eine Längsrichtung 12, ein erstes und ein zweite Ende 13, 14, sowie ein Grundkörperlumen 15 aufweist, welches sich vom ersten Ende 13 bis zum zweiten Ende 14 erstreckt. Ferner umfasst das Gefäßimplantat 10 einen ersten Endbereich 16 im Bereich des ersten Endes 13, sowie einen zweiten Endbereich 17 im Bereich des zweiten Endes 14. Dabei ist das erste Ende 13 vorzugsweise das proximale Ende 13' und das zweite Ende 14 das distale Ende 14', mit einem proximalen Endbereich 16' und einem distalen Endbereich 17'.

Wie Fig. 1 zu entnehmen ist, wird der hohlzylindrische Grundkörper der in Fig.1 gezeigten Ausführungsform des Gefäßimplantats 10 durch ein röhrchenförmiges Gittergerüst 18 gebildet, welches gemäß bevorzugten Ausführungsformen mit einem Gefäßimplantatmaterial bedeckt sein kann, bzw. an welches das Gittergerüst 18 angebracht, bspw. angenäht oder angeklebt, sein kann. Das Gefäßimplantat 10 in Fig. 1 kann dabei zu ca. 30, 50 oder zu 100% mit dem Gefäßimplantatmaterial gecovert sein.

Das Gittergerüst 18 der in der Fig. 1 gezeigten ersten Ausführungsform des Erfindungsgemäßen Gefäßimplantats 10 besteht aus in seiner Längsrichtung 12 hintereinander angeordneten Stentringen bzw. Stentfedern 21 aus mäanderförmig umlaufenden Spitzbögen, die jeweils aus zwei Schenkeln mit dazwischen liegenden Scheitel- bzw. Tiefstpunkten gebildet werden.

Zur Ausbildung von gecoverten Gefäßimplantaten können die Stentringe/Stentfedern 21 auf der Außenseite des hohlzylindrischen Körpers 11 durch ein an den Stentringen bzw. Stentfedern 21 befestigtes Implantatmaterial miteinander verbunden sein. Dabei überschneiden sich aber die einzelnen Stentringe/Stentfedern 21 im gevocerten Abschnitt nicht und sind auch sonst nicht über etwas Verbindungsstege miteinander verbunden, sondern stehen nur über das Implantatmaterial miteinander indirekt "in Verbindung".

Fig. 1 ist ferner zu entnehmen, dass sich im proximalen Endbereich 16' mehr Stentringe/Stentfedern 21 befinden als im distalen Endbereich 17'. Dies wird in der in Fig. 1 gezeigten Ausführungsform dadurch erreicht, dass zum einen im proximalen Endebereich 16' die "Amplituden", mit der die Spitzbögen 21 der Stentringe/Stentfedern 21 ausschlagen, kürzer und nicht so hoch sind, wie die der Stentringe/Stentfedern 21 im distalen Endbereich 17'. Auch umfassen die Stentringe/Stentfedern 21 im proximalen Endbereich 16' mehr Spitzbögen als die die Stentringe/Stentfedern 21 im distalen Endbereich 17'.

Dadurch ergibt sich im proximalen Endbereich 16' eine insgesamt höhere Dichte an Stentringmaterial wodurch in diesem Bereich eine höhere Radialkraft erreicht wird. Dies ist auch durch die unter dem Gefäßimplantat 10 dargestellten Pfeile verdeutlicht, mittels welchen die Verteilung der Radialkräfte (hoch -> niedrig) und die Stärke der Steifigkeit (hoch -> niedrig) über die Längsrichtung des Gefäßimplantats dargestellt ist.

Das in Fig. 1 und allen anderen Figuren dargestellte erfindungsgemäße Gefäßimplantat 10 besitzt vorzugsweise eine Radialkraft, die vom proximalen Endbereich 16' (hoch) zum distalen Endbereich 17 kontinuierlich abnimmt, und eine Weichheit die vom proximalen Endbereich 16' zum distalen Endbereich 17' kontinuierlich zunimmt. Entsprechend besitzt das distale Ende 16 bzw. der distale Endberiech 16' eine niedrige Radialkraft, aber eine hohe Weichheit, und ist daher insbesondere für Gefäßabschnitte vorgesehen, die aufgrund ihrer Dünnwandigkeit empfindlich sind.

Fig. 2 zeigt eine schematische, nicht-maßstabsgetreue Darstellung eines nicht zur Erfindung gehörenden Gefäßimplantats 10', wobei hier die gleichen Merkmale wie die des Gefäßimplantats aus Fig. 1 mit den gleichen Bezugszeichen versehen sind.

Entsprechend besitzt auch das nicht zur Erfindung gehörende Gefäßimplantat 10' einen hohlzylindrischen Grundkörper 11, sowie eine Längsrichtung 12, ein erstes und ein zweite Ende 13, 14, sowie ein Grundkörperlumen 15, welches sich vom ersten Ende 13 bis zum zweiten Ende 14 erstreckt. Ferner umfasst das Gefäßimplantat 20 einen ersten Endbereich 16 im Bereich des ersten Endes 13, sowie einen zweiten Endbereich 17 im Bereich des zweiten Endes 14. Dabei ist das erste Ende 13 vorzugsweise das proximale Ende 13' und das zweite Ende 14 das distale Ende 14', mit einem proximalen Endbereich 16' und einem distalen Endbereich 17'.

Wie Fig. 2 zu entnehmen ist, wird der hohlzylindrische Grundkörper 11 des in Fig.2 gezeigten nicht zur Erfindung gehörenden Gefäßimplantats 10' zwar ebenfalls durch ein röhrchenförmiges Gittergerüst 18 gebildet, welches mit einem Gefäßimplantatmaterial bedeckt sein kann, bzw. an welches das Gittergerüst 18 angebracht, bspw. angenäht oder angeklebt, sein kann. Das Gefäßimplantat 10' in Fig. 2 kann dabei ebenfalls zu ca. 30, 50 oder zu 100% mit dem Gefäßimplantatmaterial gecovert sein. Andererseits ist das Gittergerüst 18 des in Fig. 2 gezeigten Gefäßimplantats 10' durch eine Flechtstruktur bewirkt, und zwar indem sich eine Vielzahl von fadenförmigen Elementen in einer Ebene senkrecht zur Längsrichtung des Gefäßimplantats 10' und unter einem Flechtwinkel schneiden, und Maschen bilden. Die Maschendichte ist dabei im proximalen Endbereich 16' höher und die Maschengröße kleiner als jeweils im distalen Endbereich 17'.

Dies höhere Dichte und kleinere Maschengröße im proximalen Endbereich 16' bewirkt dabei eine höhere Radialkraft und geringere Weichheit bzw. eine größere Steifheit in diesem Bereich als es im distalen Endbereich 17' der Fall ist, in welchem eine größere Maschengröße und damit eine kleinere Maschendichte vorliegt.

Bei der in Fig. 2 gezeigten Flechtstruktur kann auch vorgesehen sein, dass sich die die Flechtstruktur bildenden Filamente in einem Flechtwinkel schneiden, der am proximalen Endbereich kleiner ist als am distalen Endbereich, und wobei sich der Flechtwinkel vom proximalen Ende zum distalen Ende kontinuierlich vergrößert.

Auch bei einer Flechtstruktur kann ein kontinuierlicher Verlauf der Radialkraft, nämlich deren kontinuierliche Abnahme vom proximalen 16' zum distalen Endbereich 17' erreicht werden.

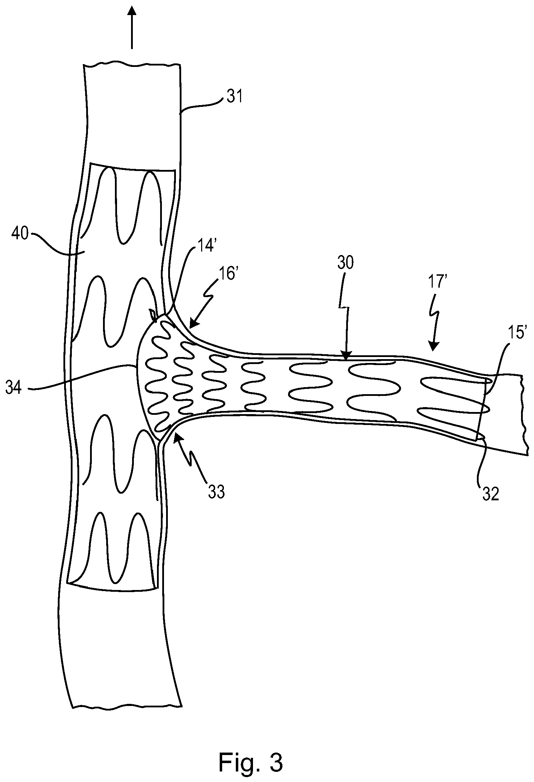

Fig. 3 zeigt schließlich die schematische Darstellung einer in ein abzweigendes Gefäß eingebrachten Ausführungsform des erfindungsgemäßen Gefäßimplantats 30. In Fig. 3 ist mit 31 ein Haupt(blut)gefäß bezeichnet, von dem ein Seitengefäß 32 an der Stelle 33 abzweigt. In das Hauptblutgefäß 31 ist an der Abzweigungsstelle 33 ein Stentgraft 40 eingebracht, das bspw. zur Offenhaltung des Hauptblutgefäßes 31, oder zur Überbrückung eines Aneurysmas, o.a., in das Hauptblutgefäß 31 platziert wurde. Um den Blutfluss in das Seitengefäß 32 nicht zu blockieren, muss über den Stentgraft 40 ein Seitenast angebracht werden, der das erfindungsgemäße Gefäßimplantat 30 darstellt. Dieses kann bspw. über eine im Stentgraft 40 vorgesehene Fenestrierung 34 im/am Stentgraft 40 fixiert werden. Dabei ist es wichtig, dass im Bereich des Übergangs bzw. der Fixierung von Stentgraft 40 zum Seitenast/Gefäßimplantat 30 eine hohe Radialkraft im Gefäßimplantat 30 vorliegt, um zwischen den beiden Implantaten 30, 40 eine stabile Verbindung herzustellen und um die Abzweigungsstelle 33 sicher abzudichten. Daher weist das Gefäßimplantat 30 mit seinem proximalen Ende 16, dessen proximaler Endbereich 16' eine höhere Radialkraft aufweist, in die Fenestrierung 34 des Stentgrafts 40 eingebracht, wobei der restliche Grundkörper 11 des Gefäßimplantats 30 in das Seitengefäß 32 hineinführt und dort mit seinem distalen Ende 17 und distalen Endbereich 17' endet.

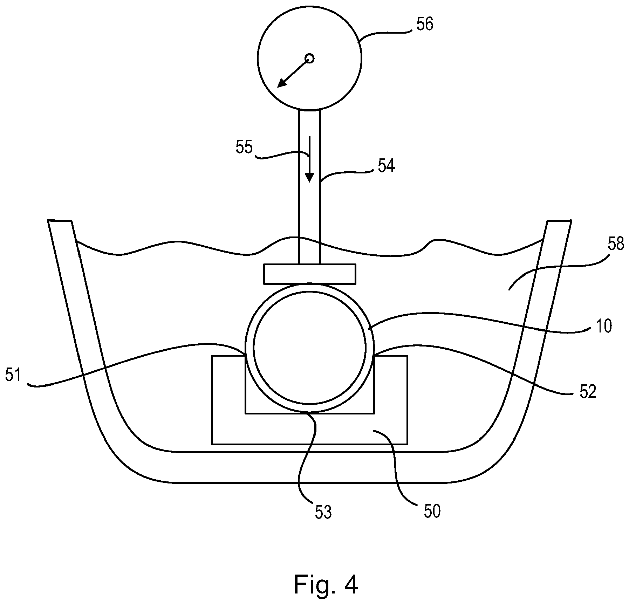

Fig. 4 zeigt schließlich eine schematische Darstellung eines Versuchsaufbaus zur Messung der Radialkraft eines erfindungsgemäßen Gefäßimplantats 10, 30: Für die Messung der Radialkraft eines Gefäßimplantats wird eine Aufnahme 50 bereitgestellt, die entweder U-förmig, eckig oder V-förmig ausgebildet sein kann. In der in Fig. 4 gezeigten Darstellung ist die Aufnahme klammerförmig eckig. In diese Aufnahme 50 wird das zu untersuchende Gefäßimplantat 10, 30 eingebracht, so dass dieses an drei Punkten 51, 52, und 53 darin fixiert ist.

Mittels eines Stempels 54 wird Druck (dargestellt durch den Pfeil 55) auf das in der Aufnahme 50 vorliegende Gefäßimplantat 10, 30 aufgebaut und dieses um einen bestimmten Prozentsatz seines Durchmessers komprimiert, bspw. um 30% seines Durchmessers. Über eine an den Stempel 54 angeschlossene Messdose 56 kann dann der zur Komprimierung des Gefäßimplantats bzw. von dessen bestimmten Bereichen aufgebrachte Druck gemessen werden, welcher der Radialkraft des Gefäßimplantats 10, 30 entspricht.

In Fig. 4 ist ferner dargestellt, dass der Versuchsansatz zur Messung der Radialkraft, vielmehr die Aufnahme 50 und das darin aufgenommene Gefäßimplantat 10 in einem Wasserbad 58 gelagert sind; das Wasserbad hat vorzugsweise KörperTemperatur (37°C), um die Radialkraft des Gefäßimplantats 10, 30 unter *in-vivo-*Bedingungen messen zu können.

## Patentansprüche

1. Gefäßimplantat (10; 30) zur Implantation in Gefäß eines Patienten, wobei das Gefäßimplantat (10; 30) von einem komprimierten Zustand in einen expandierten Zustand überführbar ist, mit einem hohlzylindrischen Grundkörper (11) mit einer Längsrichtung (12) sowie mit einem ersten (13) und einem zweiten Ende (14) und ersten (16) und zweiten Endbereichen (17), und einem Grundkörperlumen (15), das sich vom ersten (13) bis zum zweiten Ende (14) erstreckt, wobei der hohlzylindrische Grundkörper (11) durch ein röhrchenförmiges Gittergerüst (18) gebildet wird, wobei das Gefäßimplantat (10; 30) zumindest einen ersten härteren Bereich mit einer Radialkraft und einer Steifigkeit aufweist, die höher ist als die Radialkraft und Steifigkeit zumindest eines zum ersten härteren Bereich benachbarten zweiten weicheren Bereichs ist, wobei das Gefäßimplantat ferner über seine Längsrichtung (12) einen kontinuierlichen Übergang von der höheren Steifigkeit und Radialkraft im ersten härteren Bereich zur niedrigeren Steifigkeit und Radialkraft im zweiten weicheren Bereich aufweist, und wobei das Gefäßimplantat (10; 30) zumindest einen Bereich aufweist, der ein mit dem Gittergerüst (18) befestigtes Implantatmaterial aufweist, und wobei das erste Ende (13) das proximale Ende ist, und das zweite Ende (14) das distale Ende, wobei der erste härtere Bereich im proximalen Endbereich gebildet ist, und der zweite weichere Bereich im distalen Endbereich, **dadurch gekennzeichnet, dass** das röhrchenförmige Gittergerüst (18) gebildet ist aus hintereinander angeordneten und senkrecht zur Längsrichtung jeweils mäanderförmig umlaufenden einstückigen Stentfedern (21), die abwechselnd zum proximalen und distalen Ende des Grundkörpers (11) und parallel zu dessen Längsrichtung (12) weisende Spitzbögen aufweisen, wobei ein Spitzbogen aus jeweils einem Scheitelpunkt und zwei Schenkeln gebildet ist, wobei die Stentfedern (21) im proximalen Endbereich jeweils mehr Spitzbögen aufweisen als die Stentfedern (21) im distalen Endbereich.

2. Gefäßimplantat (10; 30) nach Anspruch 1, **dadurch gekennzeichnet, dass** jeder Bereich des Gefäßimplantats (10; 30) ein mit dem Gittergerüst (18) befestigtes Gefäßimplantatmaterial aufweist.

3. Gefäßimplantat (10; 30) nach Anspruch 1, **dadurch gekennzeichnet, dass** die Schenkel der Spitzbögen im distalen Endbereich eine Länge aufweisen, die größer ist als die Länge der Schenkel der Spitzbögen (21) im proximalen Endbereich

4. Gefäßimplantat (10; 30) nach einem der Ansprüche 1 bis 3, **dadurch gekennzeichnet, dass** das Implantatmaterial, vom proximalen Endbereich aus beginnend, mit dem Gittergerüst (18) über eine Fläche verbunden ist, die ausgewählt ist aus mind. ca. 30%, 50% oder 100% der Gesamtfläche des Gefäßimplantats (10; 30).

5. Gefäßimplantat (10; 30) nach einem der Ansprüche 1 bis 4, **dadurch gekennzeichnet, dass** das das Gittergerüst (18) ein Material aufweist, das ausgewählt ist aus Nitinol oder Stahl.

6. Gefäßimplantat (10; 30) nach einem der Ansprüche 1 bis 5, **dadurch gekennzeichnet, dass** das Gittergerüst (18) Nitinol aufweist oder aus diesem gebildet ist, und von einem nicht expandierten in einen selbstexpandierten Zustand überführt werden kann, und wobei das Gefäßimplantat (10; 30) im selbstexpandierten Zustand ein aufgeweitetes proximales Ende aufweist.

7. Gefäßimplantat (10; 30) nach einem der Ansprüche 1 bis 5, **dadurch gekennzeichnet, dass** das Gittergerüst (18) Stahl aufweist oder aus diesem gebildet ist, und dass das proximale Ende über einen expandierbaren Ballon von einem nicht-expandierten Zustand in einen ballon-expandierten Zustand aufweitbar ist.

8. Gefäßimplantat (10; 30) nach einem der Ansprüche 1 bis 7, **dadurch gekennzeichnet, dass** das Gefäßimplantat ein gelaserter Nitinolstent ist, und in dem zumindest einen härteren Bereich einen Wert von ca. zwischen 2,00 und 4,00 N (Newton) besitzt und in dem zumindest einen weicheren zweiten Bereich einen Wert von ca. zwischen 0,50 und 2,00 N (Newton) besitzt.

9. Gefäßimplantat (10; 30) nach einem der Ansprüche 1 bis 7, **dadurch gekennzeichnet, dass** das röhrchenförmige Gittergerüst (18) ein gelaserter Stahlstent ist, und in dem zumindest einen härteren Bereich einen Wert von ca. zwischen 5,00 und 10,00 N (Newton) besitzt und in dem zumindest einen weicheren zweiten Bereich einen Wert von ca. zwischen 1,25 und 5,00 N (Newton) besitzt.

10. Gefäßimplantat (10; 30) nach einem der Ansprüche 1 bis 9, **dadurch gekennzeichnet, dass** es zur Einbringung in ein von dem Gefäß (31) abzweigendes Seitengefäß (32) ausgebildet ist, wobei der proximale Endbereich vorgesehen ist, über eine Fenestrierung eines im Gefäß positionierten Hauptgefäßimplantats an der Abzweigung des Seitengefäßes positioniert zu werden, und der distale Endbereich im abzweigenden Seitengefäß positionierbar ist.

## Claims

1. Vascular implant (10; 30) for implantation in the vessel of a patient, the vascular implant (10; 30) being transferable from a compressed state to an expanded state, and comprising a hollow cylindrical body (11) having a longitudinal direction (12) and a first (13) and second (14) end, and first (16) and second (17) end regions, and a body lumen (15) extending from said first (13) to said second (14) end, wherein the hollow cylindrical body (11) is formed by a tubular lattice framework (18), wherein the vascular implant (10; 30) comprises at least one first harder region having a radial force and a stiffness higher than the radial force and stiffness of at least one second softer region adjacent to the first harder region, wherein the vascular implant, along its longitudinal direction (12), further comprises a continuous transition from the higher stiffness and radial force in the first harder region to the lower stiffness and radial force in the second softer region, and wherein the vascular implant (10) has at least one region comprising an implant material secured to the lattice framework (18), and wherein the first end (13) represents the proximal end and the second end (14) represents the distal end, wherein the first harder region is formed in the proximal end region, and the second softer region is formed in the distal end region, **characterized in that** the tubular lattice framework (18) is formed of one-piece stent springs (21) arranged one behind the other and circumferentially meandering perpendicularly to the longitudinal direction, which stent springs have pointed arcs pointing alternately to the proximal and distal ends of the body (11) and parallel to the longitudinal direction (12) thereof, wherein an pointed arc is formed from an apex and two legs, wherein the stent springs (21) in the proximal end region each have more pointed arcs than the stent springs (21) in the distal end region.

2. The Vascular implant (10; 30) according to claim 1, **characterized in that** each region of the vascular implant (10; 30) comprises a vascular implant material secured to the lattice framework (18).

3. The vascular implant (10; 30) according to claim 1, **characterized in that** the legs of the pointed arches in the distal end region have a length which is greater than the length of the legs of the pointed arches in the proximal end region.

4. The vascular implant (10; 30) according to any of claims 1 to 3, **characterized in that** the implant material, starting from the proximal end region, is connected to the lattice framework (18) via an area selected from at least about 30%, 50% or 100% of the total area of the vascular implant (10; 30).

5. The vascular implant (10; 30) according to any one of claims 1 to 4, **characterized in that** said lattice framework (18) comprises a material selected from nitinol or steel.

6. The vascular implant (10; 30) according to any of claims 1 to 5, **characterized in that** the lattice framework (18) comprises or is formed from nitinol and can be transferred from an unexpanded to a self-expanded state, and wherein the vascular implant (10; 20; 30) in the self-expanded state has an expanded proximal end.

7. The vascular implant (10; 30) according to any one of claims 1 to 5, **characterized in that** the lattice framework (18) comprises or is formed from steel and **in that** the proximal end is expandable via an expandable balloon from a non-expanded state to a balloon-expanded state.

8. The vascular implant (10; 30) according to any of claims 1 to 7, **characterized in that** the vascular implant is a lasered nitinol stent and has a value of about between 2.00 and 4.00 N (Newton) in the at least one harder region and a value of about between 0.50 and 2.00 N (Newton) in the at least one softer second region.

9. The vascular implant (10; 30) according to any one of claims 1 to 7, **characterized in that** the tubular lattice framework (18) is a lasered steel stent and has a value of about between 5.00 and 10.00 N (Newton) in the at least one harder region and a value of about between 1.25 and 5.00 N (Newton) in the at least one softer second region.

10. The vascular implant (10; 30) according to any one of claims 1 to 9, **characterized in that** it is designed for insertion into a side vessel (32) branching off from the vessel (31), wherein the proximal end region is provided to be positioned at the branching off of the side vessel via a fenestration of a main vessel implant positioned in the vessel, and the distal end region can be positioned in the branching off side vessel.

## Revendications

1. Implant vasculaire (10 ; 30) pour l'implantation dans un vaisseau d'un patient, l'implant vasculaire (10 ; 30) pouvant être transféré d'un état comprimé à un état expansé, comprenant un corps de base cylindrique creux (11) ayant une direction longitudinale (12) ainsi qu'une première (13) et une deuxième (14) extrémité et des premières (16) et deuxièmes (17) régions d'extrémité, et une lumière de corps de base (15) qui s'étend depuis la première (13) jusqu'à la deuxième (14) extrémité, le corps de base cylindrique creux (11) étant formé par une structure maillée (18) en forme de petits tubes, l'implant vasculaire (10 ; 30) présentant au moins une première région plus dure avec une force radiale et une rigidité supérieures à la force radiale et à la rigidité d'au moins une deuxième région plus souple adjacente à la première région plus dure, l'implant vasculaire présentant en outre sur sa direction longitudinale (12) une transition continue de la rigidité et de la force radiale plus élevées dans la première région plus dure à la rigidité et à la force radiale plus faibles dans la deuxième région plus souple et l'implant vasculaire (10 ; 30) présentant au moins une région qui présente un matériau d'implant fixé avec la structure maillée (18), et la première extrémité (13) étant l'extrémité proximale, et la deuxième extrémité (14) étant l'extrémité distale, la première région plus dure étant formée dans la région d'extrémité proximale et la deuxième région plus souple étant formée dans la région d'extrémité distale, **caractérisé en ce que** la structure maillée (18) en forme de petits tubes est formée de ressorts d'endoprothèse (21) d'une seule pièce disposés les uns derrière les autres et s'étendant à chaque fois sur la circonférence en forme de méandres perpendiculairement à la direction longitudinale, qui présentent des arcs à angle aigu orientés en alternance vers l'extrémité proximale et vers l'extrémité distale du corps de base (11) et orientés parallèlement à sa direction longitudinale (12), un arc à angle aigu étant formé à chaque fois d'un sommet et de deux branches, les ressorts d'endoprothèse (21) présentant dans la région d'extrémité proximale à chaque fois plus d'arcs à angle aigu que les ressorts d'endoprothèse (21) dans la région d'extrémité distale.

2. Implant vasculaire (10 ; 30) selon la revendication 1, **caractérisé en ce que** chaque région de l'implant vasculaire (10 ; 30) présente un matériau d'implant vasculaire fixé avec la structure maillée (18).

3. Implant vasculaire (10 ; 30) selon la revendication 1, **caractérisé en ce que** les branches des arcs à angle aigu dans la région d'extrémité distale présentent une longueur supérieure à la longueur des branches des arcs à angle aigu (21) dans la région d'extrémité proximale.

4. Implant vasculaire (10 ; 30) selon l'une quelconque des revendications 1 à 3, **caractérisé en ce que** le matériau d'implant, en commençant à partir de la région d'extrémité proximale, est connecté à la structure maillée (18) par le biais d'une surface qui est choisie à partir d'au moins environ 30 %, 50 % ou 100 % de la surface totale de l'implant vasculaire (10 ; 30).

5. Implant vasculaire (10 ; 30) selon l'une quelconque des revendications 1 à 4, **caractérisé en ce que** la structure maillée (18) présente un matériau choisi parmi le nitinol ou l'acier.

6. Implant vasculaire (10 ; 30) selon l'une quelconque des revendications 1 à 5, **caractérisé en ce que** la structure maillée (18) présente du nitinol ou est formée de celui-ci, et peut être transférée d'un état non expansé à un état auto-expansé, et l'implant vasculaire (10 ; 30), dans l'état auto-expansé, présentant une extrémité proximale élargie.

7. Implant vasculaire (10 ; 30) selon l'une quelconque des revendications 1 à 5, **caractérisé en ce que** la structure maillée (18) présente de l'acier ou est formée de celui-ci, et **en ce que** l'extrémité proximale peut être élargie par le biais d'un ballonnet expansible d'un état non expansé à un état expansé par le ballonnet.

8. Implant vasculaire (10 ; 30) selon l'une quelconque des revendications 1 à 7, **caractérisé en ce que** l'implant vasculaire est une endoprothèse de nitinol traitée au laser, et dans lequel au moins une région plus dure possède une valeur comprise entre environ 2,00 et 4,00 N (Newton) et dans lequel au moins une deuxième région plus souple possède une valeur comprise entre environ 0,50 et 2,00 N (Newton).

9. Implant vasculaire (10 ; 30) selon l'une quelconque des revendications 1 à 7, **caractérisé en ce que** la structure maillée (18) en forme de petits tubes est une endoprothèse d'acier traitée au laser, et dans lequel au moins une région plus dure possède une valeur comprise entre environ 5,00 et 10,00 N (Newton) et dans lequel au moins une deuxième région plus souple possède une valeur comprise entre environ 1,25 et 5,00 N (Newton).

10. Implant vasculaire (10 ; 30) selon l'une quelconque des revendications 1 à 9, **caractérisé en ce qu'**il est réalisé pour être introduit dans un vaisseau latéral (32) partant du vaisseau (31), la région d'extrémité proximale étant prévue pour être positionnée, par le biais d'une fenestration d'un implant de vaisseau principal positionné dans le vaisseau, au niveau du branchement du vaisseau latéral et la région d'extrémité distale pouvant être positionnée dans le vaisseau latéral de branchement.
